(19) Europäisches Patentamt / European Patent Office / Office européen des brevets

(11) **EP 3 107 061 B1**

(12) **EUROPEAN PATENT SPECIFICATION**

(45) Date of publication and mention
of the grant of the patent:
**02.12.2020 Bulletin 2020/49**

(21) Application number: **14882248.9**

(22) Date of filing: **04.04.2014**

(51) Int Cl.:
**G16H 10/20** (2018.01)      **G16H 50/30** (2018.01)
**G16H 50/20** (2018.01)      **G16H 40/67** (2018.01)

(86) International application number:
**PCT/JP2014/059931**

(87) International publication number:
**WO 2015/122026 (20.08.2015 Gazette 2015/33)**

(54) **DISEASE DETECTION SYSTEM AND DISEASE DETECTION METHOD**

KRANKHEITSERKENNUNGSSYSTEM UND KRANKHEITSERKENNUNGSVERFAHREN

SYSTÈME DE DÉTECTION DE MALADIE ET PROCÉDÉ DE DÉTECTION DE MALADIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.02.2014 JP 2014024165**

(43) Date of publication of application:
**21.12.2016 Bulletin 2016/51**

(73) Proprietor: **Shimura, Akiyoshi
Tokyo 158-0083 (JP)**

(72) Inventor: **Shimura, Akiyoshi
Tokyo 158-0083 (JP)**

(74) Representative: **Vossius & Partner
Patentanwälte Rechtsanwälte mbB
Siebertstrasse 3
81675 München (DE)**

(56) References cited:
**WO-A1-2011/016447      JP-A- 2008 158 748
JP-A- 2012 221 447      JP-A- 2013 076 597
US-A1- 2004 243 443      US-A1- 2005 131 695
US-A1- 2005 137 463**

# EP 3 107 061 B1

**Description**

## TECHNICAL FIELD

The present invention relates to a disease detecting system and a disease detecting method, and more particularly, is suitably used for a system serving to access a server on internet from a user terminal, thereby detecting a possibility of a disease.

## BACKGROUND ART

Conventionally, there is known a system for practically using the IT (Information Technology) to detect a disease (for example, see Patent Documents 1 to 3). The system described in the Patent Document 1 analyzes a language of a free sentence describing observation of a doctor or the like, thereby detecting a disease condition phrase. In the system described in the Patent Document 2, moreover, a group of non-stereotyped sentences is divided into a word or a phrase by using a natural language analyzing technique based on diagnostic text information associated with a pathological specimen image, and a conversion dictionary for a relationship between a disease and a keyword is created by using a technique for analyzing their appearance frequency or correlation to extract useful information.

Moreover, the system described in the Patent Document 3 diagnoses a disease condition from a patient database for storing data to be used for diagnosing a disease condition of an impatient for a completely physical examination, a knowledge database for setting an item of the patient database and an attribute thereof as a condition part and storing a cause and effect relationship with a diagnostic result set to be a conclusion part, and a comment database for storing data on a comment item, for example, explanation knowledge and observation knowledge possessed by a doctor for the patient database, instructions and precautions given to a patient, and the like.

The patient database is obtained by storing, together with a patient ID code, data to be used for diagnosing a disease condition of an impatient for a completely physical examination including test result data acquired by storing a test result such as a blood test or a biochemical test, medical examination result data acquired by storing a diagnostic result obtained through a specimen test such as electrocardiography or abdominal ultrasonography, measurement result data obtained by storing a measurement result such as a height, a weight, a blood pressure or a pulse, and medical interview result data obtained by storing a medical interview result such as a complaint of a patient or an everyday life style.

Patent Document 1 : Japanese Laid-Open Patent Publication No. 2008-108199
Patent Document 2 : Japanese Laid-Open Patent Publication No. 2012-179336
Patent Document 3 : Japanese Laid-Open Patent Publication No. Hei 6-176007

US 2005/0137463 A1 relates to a system and method for generating a medical diagnosis. A conversion table is created and stored in a computerized storage media of a computerized system, wherein the conversion table converts raw test data into numeric values. Furthermore, a sub-diagnosis database is created and stored in the storage media, the sub-diagnosis database including a plurality of rules, each rule being identified by at least one diagnosis parameter. Patient test results are then input to the computerized system where they are converted into numeric analyzed values by the conversion table. The numeric analyzed values are compared to the diagnosis parameters of the rules stored in the sub-diagnosis database, wherein the rules having diagnosis parameters corresponding to the numeric analyzed values are saved in the computerized system.

## DISCLOSURE OF THE INVENTION

The invention is defined by independent claims 1 and 6. Preferred embodiments are defined by dependent claims 2 to 5.

All of the systems described in the Patent Documents 1 to 3 detect a disease or a disease condition based on text information such as an observation or a comment created by a doctor when a patient gets a medical examination from the doctor. For a general user who has not got the medical examination from the doctor yet, therefore, it is impossible to previously detect a disease having an affection doubt. In other words, the system cannot be used in a disease self-check system for the general public which is intended for preventive medicine.

Conventionally, there is also provided a system for a general user to answer a question of a medical interview sheet opened on internet depending on a subjective symptom, thereby detecting a disease which might be affected based on the content of the answer. Referring to the system of this type, however, there is fixed an algorithm for detecting any kind of contents of an answer to a prepared question that has a doubt of a possibility of affection with any disease. However, the detection algorithm is usually generated based on an experimental rule of a specific doctor and is not always optimum.

In order to solve the problem, it is an object of the present invention to enable detection, with higher precision, a disease doubted to be affected for a general user who is not diagnosed by a doctor.

In order to attain the object, the present invention uses profile data of a user, medical interview answer data to be an answer given from the user to a medical interview sheet, text related data obtained by analyzing free answer data to be an answer from the user to a question in a free sentence description format, and a predetermined weight value to analyze an affection probability representing a possibility that the user might be affected with a specific disease and to extract, as a disease candidate, a disease having the affection probability which is equal to or greater than a threshold. Moreover, the profile data, the medical interview answer data and the text related data are stored and accumulated in a database at any time, and the profile data, the medical interview answer data and the text related data in the database are set to be explanatory variables and the extracted disease candidate is set to be a criterion variable, thereby performing regression analysis. Consequently, a weight value is calculated for an explanatory variable to be a factor correlated with a disease candidate extracted in this analysis and is used for analyzing an affection probability at a next time and thereafter.

According to the present invention having the structure described above, an affection probability representing a possibility that a user might be affected with a specific disease is not calculated by either only the medical interview answer data to the medical interview sheet or only the free answer data to the question which can be answered in a free sentence but by both of them. In addition, when the affection probability is to be calculated, the weight value calculated with respect to the factor to be the cause of the extraction as the disease candidate with the affection probability of a threshold or more in past analysis is reflected in the calculation of the affection probability. Therefore, the affection probability can be calculated more accurately. The weight value is more statistically significant with an increase in the number of analyzing operations for the affection probability. Therefore, precision in the analysis of the affection probability is gradually enhanced. Consequently, it is possible to detect, with higher precision, a disease having a possibility that a general user who is not diagnosed by a doctor might be affected.

## BRIEF DESCRIPTION OF THE DRAWINGS

FIG. 1 is a diagram showing an example of a whole structure of a disease detecting system according to the present embodiment.

FIG. 2 is a block diagram showing an example of a functional structure of a disease detecting server according to the present embodiment.

FIG. 3 is a flowchart showing an example of an operation of the disease detecting server according to the present embodiment.

FIG. 4 is a diagram showing another example of the structure of the disease detecting system according to the present embodiment.

## BEST MODE FOR CARRYING OUT THE INVENTION

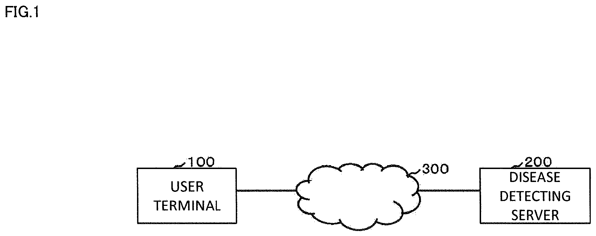

An embodiment according to the present invention will be described below with reference to the drawings. FIG. 1 is a diagram showing an example of a whole structure of a disease detecting system according to the present embodiment. As shown in FIG. 1, the disease detecting system according to the present embodiment includes a user terminal 100 and a disease detecting server 200 and has a structure in which the user terminal 100 and the disease detecting server 200 can be connected to each other through a communication network such as internet 300. The user terminal 100 is used by a general user subjected to detection of a disease online. Actually, a plurality of user terminals 100 is present, which is simplified in FIG. 1.

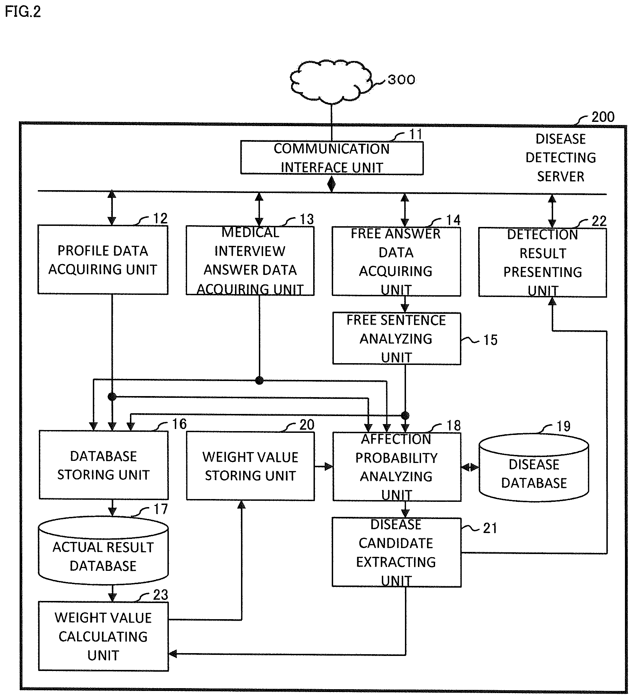

FIG. 2 is a block diagram showing an example of a functional structure of the disease detecting server 200 according to the present embodiment. As shown in FIG. 2, the disease detecting server 200 according to the present embodiment includes, as a functional structure thereof, a communication interface unit 11, a profile data acquiring unit 12, a medical interview answer data acquiring unit 13, a free answer data acquiring unit 14, a free sentence analyzing unit 15, a database storing unit 16, a database 17, an affection probability analyzing unit 18, a disease database 19, a weight value storing unit 20, a disease candidate extracting unit 21, a detection result presenting unit 22 and a weight value calculating unit 23.

Each of the functional blocks 11 to 23 can also be configured from any of hardware, a DSP (Digital Signal Processor) and software. For example, in the case in which each of the functional blocks 11 to 23 is configured from the software, it actually includes a CPU, an RAM, an ROM and the like of a computer and is implemented by an operation of a program stored in a recording medium such as the RAM, the ROM, a hard disk, a semiconductor memory or the like.

The communication interface unit 11 performs a bidirectional communication together with the user terminal 100 through the internet 300, thereby transmitting and receiving information. The profile data acquiring unit 12 acquires profile data representing a profile of a user (an age, a gender, an address, an occupation, a life style or the like). For example, when the disease detecting server 200 is accessed by the user terminal 100, the profile data acquiring unit 12 presents a predetermined profile input screen to the user terminal 100 and acquires the profile data of the user input from the profile input screen.

In the case in which the disease detecting server 200 prestores the profile data of the user as a user information database (not shown) in relation to a predetermined ID or the case in which the user information database and the disease detecting server 200 cooperate with each other, moreover, the profile data acquiring unit 12 may acquire profile data corresponding to an ID transmitted from the user terminal 100 through the user information database based on the ID.

The medical interview answer data acquiring unit 13 presents data on a medical interview sheet to the user terminal 100 and acquires medical interview answer data to be an answer to the medical interview sheet. The data on the medical interview sheet which is presented indicates data on an input screen which is expressed in a format of the medical interview sheet including a question item related to an open question which can be answered by choices and a question item related to a close question to ask sleeping hours per day or an average alcohol drinking amount per time. The user inputs an answer to each of the question items displayed on the input screen. Medical interview answer data generated by the answer is transmitted from the user terminal 100 to the disease detecting server 200 and the medical interview answer data acquiring unit 13 acquires the medical interview answer data.

Referring to a known main disease, a prevalence for each group is usually made apparent from an epidemiological study, and furthermore, a proper medical interview sheet is prepared. In the present embodiment, the medical interview sheet is utilized. As a typical medical interview sheet, a sensitivity and a specificity to a disease for each question are known. The sensitivity indicates a rate representing a positivity (an abnormal value) in an examination for a group affected with a specific disease, and the specificity indicates a rate representing a negativity (a normal value) in an exanimation for a group which is not affected with the specific disease. It is possible to calculate a disease affection probability after answering. A prevalence for each disease and a sensitivity and specificity for each question which is included in the medical interview sheet prepared for each disease are prestored as parameters to be used in the calculation of the disease affection probability in the disease database 19.

The free answer data acquiring unit 14 presents, to the user terminal 100, data on a question which can be answered in a free sentence and acquires free answer data to be an answer to a question. Data on the question presented herein is equivalent to data on an input screen which is expressed in a free sentence description format. The user answers a question displayed on the input screen in a format in which a subjective symptom or the like is described in a free sentence. Then, free answer data in a text sentence generated by the answer is transmitted from the user terminal 100 to the disease detecting server 200, and the free answer data acquiring unit 14 acquires the free answer data.

Although the description has been given to the example in which the input screen in the medical interview sheet format and the input screen in the free sentence description format are presented to the user terminal 100 separately, the present invention is not restricted thereto. For example, a single input screen including both a medical interview sheet and a free sentence description column may be presented to the user terminal 100.

The free sentence analyzing unit 15 analyzes the natural sentence of the free answer data acquired by the free answer data acquiring unit 14 for purpose of text mining. Consequently, at least one of a constitution word, word segmentation, a segment (a sentence including a subject and a predicate), information about a distance between words and text dependency information is extracted from a free sentence and is generated as text related data.

The database storing unit 16 relates the profile data acquired by the profile data acquiring unit 12, the medical interview answer data acquired by the medical interview answer data acquiring unit 13, and the text related data generated by the free sentence analyzing unit 15 to each other, and stores them in the actual result database 17. The database storing unit 16 stores the profile data, the medical interview answer data and the text related data in the actual result database 17 as required every time the profile data, the medical interview answer data and the free answer data are transmitted from the user terminals 100 to the disease detecting server 200.

The affection probability analyzing unit 18 analyzes an affection probability representing a possibility that a user might be affected with a specific disease by using the profile data acquired by the profile data acquiring unit 12, the medical interview answer data acquired by the medical interview answer data acquiring unit 13, the text related data generated by the free sentence analyzing unit 15, various parameters stored for each disease in the disease database 19, and a predetermined weight value stored in the weight value storing unit 20 (a risk ratio which will be described below).

More specifically, the affection probability analyzing unit 18 statistically calculates an affection probability (a post-examination probability) based on the following (Equation 1) in regard to all diseases registered in the disease database 19.

$$\text{Affection probability (post-examination probability) P}$$

$$= \text{pre-examination probability} \times \text{cumulative risk ratio} \cdots$$

$$\text{(Equation 1)}$$

First of all, the calculation of the pre-examination probability will be described. The pre-examination probability is calculated based on the profile data acquired by the profile data acquiring unit 12 and the various parameters stored for each disease in the disease database 19. In other words, reference is made to a profile element in which a risk ratio (an index indicative of a liability to a disease of a person having a factor of the disease as compared with a person having no factor) is apparent with statistical significance from the disease database 19 for all of the diseases registered in the disease database 19, and a known prevalence of the disease is multiplied by a risk ratio corresponding to a profile to obtain the pre-examination probability.

More specifically, in the case in which the disease database 19 stores, as a parameter, that a male has a risk ratio which is a double of that of a female with respect to a certain disease having a parameter of 3% of a whole prevalence stored in the disease database 19, for example, the pre-examination probability can be calculated to be 4% when the gender of the answerer acquired by the profile data acquiring unit 12 is a male.

If the number of people having the same profile as the answerer (that will hereinafter referred to as a target profile possessor) is sufficiently smaller than the number of populations (general populations), moreover, the pre-examination probability can simply be approximated by the multiplication of the risk ratio possessed by the profile. For example, in the case in which the disease database 19 stores, as a parameter, that a resident in the B area of the A prefecture has a double risk as compared with the general population for a disease having a parameter of 1% of a whole prevalence stored in the disease database 19, it is possible to calculate the pre-examination probability to be 2% when an address of the answerer acquired by the profile data acquiring unit 12 is the B area of the A prefecture.

The number of the populations in this case is stored in the disease database 19. On the other hand, the number of the target profile possessors can be obtained from profile data of users stored in the actual result database 17. The affection probability analyzing unit 18 decides whether a rate of the target profile possessors occupying the population is equal to or smaller than a predetermined value by referring to the actual result database 17 and the disease database 19, and performs the approximate calculation described above if the rate is equal to or smaller than the predetermined value.

In the case in which some risks correspond to the profile of the user, thus, their risk ratios are multiplied to obtain the pre-examination probability. In the case in which the rate of the number of the target profile possessors to the number of the populations is equal to or smaller than the predetermined value, a cumulative risk ratio calculated by the multiplication of the risk ratios can be expressed in the following (Equation 2).

$$\prod_{n=0}^{N} (RR(X_n)) \cdot \cdot \cdot \text{(Equation 2)}$$

In the (Equation 2), $X_n$ represents a profile item having a clear risk ratio with statistical significance. Herein, n indicates a number for identifying respective profile items and N indicates a number of the profile items. $RR(X_n)$ represents a risk ratio to the population, and the value is stored in the database 19. In the case of N = 0 (the case of a disease without a profile having a clear risk ratio with the statistical significance), however, the value in the (Equation 2) is one.

On the other hand, in the case in which the number of the target profile possessors is not sufficiently smaller than the number of the populations such as a gender or an age, next (Equation 3) is used for the $RR(X_N)$ portion of the (Equation 2). In the (Equation 3), however, $POP_n$ represents a rate of the target profile possessors occupying the population.

$$RR(X_n) / (POP_n \cdot RR(X_n) + 1 - POP_n) \quad \cdots \quad \text{(Equation 3)}$$

Next, description will be given to the calculation of the cumulative risk in the (Equation 1). The calculation of the cumulative risk includes a pattern for calculating the cumulative risk from the medical interview answer data acquired by the medical interview answer data acquiring unit 13 and a pattern for calculating the cumulative risk from the text related data generated by the free sentence analyzing unit 15.

First of all, description will be given to an example in which the cumulative risk is calculated from the medical interview answer data. For example, in the case in which the disease database 19 stores, as a parameter, a plurality of known screening indices supposed to have no correlation and to be independent of each other with respect to a certain disease and the medical interview answer data acquired by the medical interview answer data acquiring unit 13 indicates that a positive answer or a negative answer is given to a question item related to the screening indices, the cumulative risk of the disease can be calculated based on a known sensitivity and specificity stored as a parameter in the disease database 19.

In other words, a positive reaction hitting ratio PPV is expressed in the following (Equation 4), wherein prev represents a pre-examination probability, se represents a sensitivity and sp represents a specificity. Therefore, it is possible to calculate, with a division of the (Equation 4) by the pre-examination probability prev, a risk ratio of a question item to which a positive answer is given with respect to a medical interview sheet.

$$\mathrm{PPV} = \mathrm{prev} \cdot \mathrm{se} \ / \ \mathrm{prev} \cdot \mathrm{se} \cdot (1 - \mathrm{prev})(1 - \mathrm{sp})$$

$$\text{(Equation 4)}$$

More specifically, the affection probability analyzing unit 18 can express, in the following (Equation 5), a cumulative risk ratio indicative of a possibility that a disease might be positive in the case in which a positive answer is given to N question items $X_n$, wherein $\mathrm{prev}_A$ represents an affection probability of a disease A in the population calculated in the (Equation 2) or (Equation 3), $\mathrm{se}(X_n)$ represents a sensitivity of the question item $X_n$ having a sensitivity and specificity known, and $\mathrm{sp}(X_n)$ represents the specificity (n represents a number for identifying respective question items and N represents a number of the question items).

$$\prod_{n=0}^{N} \left( \frac{\mathrm{se}(X_n)}{\mathrm{prev}_A \cdot \mathrm{se}(X_n) + (1 - \mathrm{prev}_A)(1 - \mathrm{sp}(X_n))} \right) \cdots \text{(Equation 5)}$$

On the other hand, in the case in which a negative answer is given to the N question items $X_n$, it is possible to express, in the following (Equation 6), a cumulative risk indicative of a possibility that a disease might be positive.

$$\prod_{n=0}^{N} \left( \frac{1 - \mathrm{se}(X_n)}{\mathrm{prev}_A \cdot (1 - \mathrm{se}(X_n)) + (1 - \mathrm{prev}_A) \cdot \mathrm{sp}(X_n)} \right) \cdots \text{(Equation 6)}$$

Herein, the description has been given to the example in which the cumulative risk is calculated from the question item $X_n$ of the medical interview sheet. In the case in which an answerer previously takes a laboratory test such as a health examination, however, a value measured by the laboratory test may be utilized as an answer to a medical interview sheet. In this case, it is sufficient that the question item $X_n$ in the (Equation 5) and (Equation 6) is replaced with a test item of the laboratory test. Moreover, the cumulative risk ratio obtained by the (Equation 5) and (Equation 6) represents a cumulative risk ratio indicative of a possibility that a disease might be positive in the case in which positivity or negativity is acquired in the N test items $X_n$. For simplicity of the following explanation, it is assumed that the question item of the medical interview sheet is a concept including the test items of the laboratory test.

Referring to a cumulative risk which is not in a format of a question item related to an open question having a known sensitivity and specificity but is related to a close question having a known risk ratio by regression analysis or the like, moreover, a cumulative risk ratio indicative of a possibility that a disease might be positive can be expressed in the following (Equation 7), wherein $\mathrm{RR}(Y_m)$ represents a risk ratio of a question item $Y_m$ (m indicates a number for identifying each question item and M indicates a number of a question item).

$$\prod_{m=0}^{M} (\mathrm{RR}(Y_m)) \cdots \text{(Equation 7)}$$

In the case in which the number of the answers is not sufficiently smaller than the number of the populations such as a gender or an age, however, next (Equation 8) is used for the $\mathrm{RR}(Y_m)$ portion of the (Equation 7). In the (Equation 8), $\mathrm{POP}_m$ represents a rate of the answers occupying the population.

$$RR(Y_m)/(POP_m \cdot RR(Y_m) + 1 - POP_m) \quad \cdots \quad (Equation\ 8)$$

By the (Equation 5) to the (Equation 8) described above, a cumulative risk ratio $\Pi R$ in use of a question item $X_n$ having a known sensitivity and specificity and a question item $Y_m$ having a known risk ratio is expressed in the following (Equation 9), wherein positive and negative answers to the question item $X_n$ are represented by $X_n(aff)$ and $X_n(neg)$, respectively. In the case of N = 0 and M = 0, however, the cumulative risk ratio has a value of one.

$$\Pi R = \prod_{n=0}^{N}\left( \frac{se(X_{n(aff)})}{prev_A \cdot se(X_{n(aff)}) + (1-prev_A)(1-sp(X_{n(aff)}))} \right)$$
$$\cdot \prod_{n=0}^{N}\left( \frac{1-se(X_{n(neg)})}{prev_A \cdot (1-se(X_{n(neg)})) + (1-prev_A) \cdot sp(X_{n(neg)})} \right)$$
$$\cdot \prod_{m=0}^{M}(RR(Y_m)) \qquad \cdots \quad (Equation\ 9)$$

Next, description will be given to an example in which the cumulative risk ratio is calculated from the text related data generated by the free sentence analyzing unit 15. The affection probability analyzing unit 18 decides whether or not text related data having a risk ratio calculated with statistical significance is included in the text related data generated by the free sentence analyzing unit 15 with reference to the weight value storing unit 20. If the text related data is included, it is also used for the (Equation 7) to calculate the cumulative risk. The statistically significant risk ratio is calculated by the weight value calculating unit 23 as will be described below and is stored in the weight value storing unit 20 in relation to the text related data.

The disease candidate extracting unit 21 extracts a disease having an affection probability P obtained by the affection probability analyzing unit 18 which is equal to or greater than a predetermined threshold P(dif) as a disease candidate for a user (an answerer) subjected to the detection of the disease. A value of the threshold P(dif) is to be set individually depending on severity or urgency of a disease. For example, a disease having P(dif) = 75% and an affection probability P of 75% or more is extracted as a disease candidate for "a disease positivity".

The disease candidate extracting unit 21 may extract, as the disease candidate for the "disease positivity", a disease having the affection probability P obtained by the affection probability analyzing unit 18 which is equal to or greater than the first threshold P(dif) (for example, P(dif)= 75%), while it may extract, as a disease candidate for a "disease doubt", a disease having the affection probability P which is smaller than the first threshold P (dif) and is equal to or greater than a second threshold P(sus)(for example, P(sus) = 10%).

The detection result presenting unit 22 presents the disease candidate extracted by the disease candidate extracting unit 21 to the user terminal 100. Accessory information such as recommendation to receive a medical examination or a health advice may be presented together with the disease candidate. For example, the accessory information is prestored in the disease database 19 for each disease. In the case in which the disease candidate is extracted by a division into the "disease positivity" and the "disease doubt", it is preferable that contents of the accessary information should be different from each other in the case of the "disease positivity" and the case of the "disease doubt". The disease candidate is presented by the detection result presenting unit 22 so that a session related to single disease detection is ended.

After the end of the session, the weight value calculating unit 23 sets, as explanatory variables, the profile data, the medical interview answer data and the text related data stored in the actual result database 17 including data stored newly in the actual result database 17 and sets the disease candidate extracted by the disease candidate extracting unit 21 as a criterion variable, thereby performing regression analysis. In the case in which there is an explanatory variable to be a factor (serving as a risk factor) correlated statistically significantly with the disease candidate extracted by the disease candidate extracting unit 21, a weight value (a risk ratio) is calculated for the explanatory variable and is stored in the weight value storing unit 20. In the case in which the weight value has already been stored for the explanatory variable in the weight value storing unit 20, alternatively, the weight value is updated and stored.

For example, when the disease candidate is set to be "appendicitis", and presence of the appendicitis is set to be the criterion variable and the content of an answer to a medical interview sheet, the content of free description, for example, "I have a stomachache in a right and lower part", "I have a fever" or "I ate too much yesterday", a user profile or the like is set to be the explanatory variable to perform the regression analysis, presence of a phrase, for example, "I have a stomachache in a right and lower part" raises a risk of the appendicitis statistically significantly and a risk ratio can be calculated, for example, 15 times as much as an ordinary risk ratio.

Thus, the weight value (risk ratio) stored in the weight value storing unit 20 is used for calculating an affection probability in a next session. Every time the session is repeated, consequently, an ability to detect a disease candidate evolves by itself. In other words, it is possible to enhance a data volume related to a disease and statistical precision thereof by repeating the above processing for each disease detecting subject.

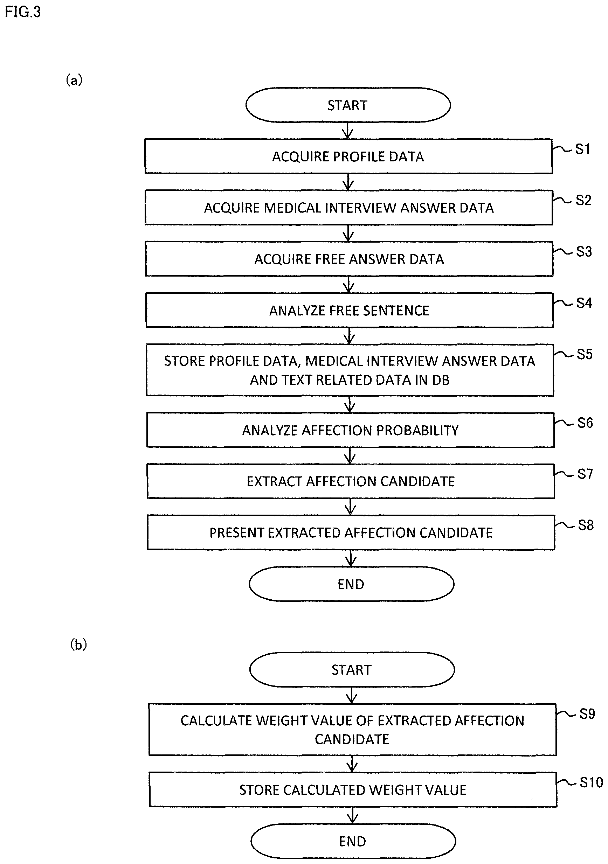

FIG. 3 is a flowchart showing an example of an operation of the disease detecting server 200 having the structure described above. A flowchart shown in FIG. 3 (a) is started when the disease detecting server 200 is accessed from the user terminal 100 in order to detect a disease and a session is established.

First of all, the profile data acquiring unit 12 acquires the profile data of the user from the user terminal 100 (Step S1). Next, the medical interview answer data acquiring unit 13 presents an input screen in a medical interview sheet format to the user terminal 100 and acquires medical interview answer data to be an answer of the user to a medical interview sheet (Step S2) . Moreover, the free answer data acquiring unit 14 presets an input screen in a free sentence description format to the user terminal 100 and acquires free answer data to be an answer of the user to a question (Step S3) .

The free sentence analyzing unit 15 analyzes the natural sentence of the free answer data acquired by the free answer data acquiring unit 14, thereby generating, as text related data, data on at least one of a constitution word, word segmentation, a segment, information about a distance between words and text dependency information (Step S4). Then, the database storing unit 16 stores, in the actual result database 17, the text related data generated by analyzing the profile data, the medical interview answer data and the free answer data of the user (Step S5).

Moreover, the affection probability analyzing unit 18 analyses the affection probability P of the user for each disease registered in the disease database 19 by using the profile data, the medical interview answer data and the text related data of the user which are acquired at this time, various parameters stored for each disease in the disease database 19, and a predetermined weight value (risk ratio) stored in the weight value storing unit 20 (Step S6).

The disease candidate extracting unit 21 extracts, as the disease candidate for "disease positivity", a disease having the affection probability P obtained by the affection probability analyzing unit 18 which is equal to or greater than the first threshold P(dif), and extracts, as the disease candidate for a "disease doubt", a disease having the affection probability P which is smaller than the first threshold P(dif) and is equal to or greater than the second threshold P (sus) (Step S7). Then, the detection result presenting unit 22 presents, to the user terminal 100, the disease candidate extracted by the disease candidate extracting unit 21 and the session is ended (Step S8).

A flowchart shown in FIG. 3 (b) is started after the end of the session. As described above, after a single session is ended by the processing of the Steps S1 to S8, the weight value calculating unit 23 sets, as explanatory variables, the profile data, the medical interview answer data and the text related data stored in the actual result database 17 and sets the extracted disease candidate as the criterion variable, thereby performing the regression analysis.

Subsequently, a weight value (risk ratio) is calculated for the explanatory variable serving as a factor to be correlated statistically significantly with the disease candidate extracted by the disease candidate extracting unit 21 (Step S9), and is updated and stored in the weight value storing unit 20 (Step S10). Consequently, the processing of the flowchart shown in FIG. 3 is ended.

As described above in detail, in the present embodiment, an affection probability representing a possibility that the user might be affected with a specific disease is analyzed to extract, as a disease candidate, a disease having the affection probability which is equal to or greater than a threshold by using the profile data of the user, the medical interview answer data to be an answer given from the user to a question in a medical interview sheet format, the text related data obtained by analyzing the free answer data to be an answer given from the user to a question in a free sentence description format, and the weight value (risk ratio) obtained by performing the regression analysis based on the actual result of the detection of the disease.

In the present embodiment, every time an answer is given from the user, the profile data, the medical interview answer data and the text related data are stored and accumulated in the actual result database 17 at any time. Then, the profile data, the medical interview answer data and the text related data in the actual result database 17 are set to be the explanatory variables including the data acquired in this analysis, and the disease candidate extracted by the disease candidate extracting unit 21 is set to be the criterion variable to perform the regression analysis. Consequently, the weight value is calculated for the explanatory variable serving as the factor to be correlated with the disease candidate extracted by this analysis and is used for analyzing the affection probability at a next time and thereafter.

According to the present embodiment thus configured, the affection probability representing the possibility that the user is affected with a specific disease is calculated by using both the medical interview answer data and the free answer data. In addition, when the affection probability is to be calculated, the risk ratio calculated in relation to the factor to be the cause of the extraction as the disease candidate with the affection probability of a threshold or more in past analysis is reflected in the calculation of the affection probability. Therefore, the affection probability can be calculated more accurately. The risk ratio has a more statistically significant value with an increase in the number of analyzing operations for the affection probability. Therefore, precision in the analysis of the affection probability is enhanced. Consequently, it is possible to detect a disease having the affection doubt with higher precision for a general user who is not diagnosed by a doctor.

In the embodiment, the following function may further be added. For example, in the case in which a disease candidate for a "disease positivity" is extracted by the disease candidate extracting unit 21, the free answer data acquiring unit 14 presents, to the user terminal 100, data on an additional question for demanding a detailed answer in a free sentence related to a disease extracted as the disease candidate, and acquires free answer data to the additional question. In this case, it is preferable to present characteristics or symptoms of the extracted disease to the user terminal 100 and to demand a detailed additional answer such as a subjective symptom or a background for them.

The free sentence analyzing unit 15 further analyses the additional free answer data acquired by the free answer data acquiring unit 14 and generates, as the text related data, data related to at least one of a constitution word, word segmentation, a segment, information about a distance between words and text dependency information. Then, the database storing unit 16 adds the text related data generated additionally by the free sentence analyzing unit 15 to the actual result database 17 and stores the added data therein. Such processing is repeated by the number of the disease candidates extracted as the "disease positivity".

By providing such additional function, it is possible to store, in the actual result database 17, text related data which is tightly related to a disease to be positive. As a result, the risk ratio is obtained for the text related data having tight relation to the disease and is stored in the weight value storing unit 20 by the regression analysis through the weight value calculating unit 23, and can be utilized for analyzing the affection probability at a next time and thereafter. Consequently, the disease probability can be calculated more accurately.

Moreover, the following function may further be added. For example, in the case in which the disease candidate for the "disease doubt" is extracted by the disease candidate extracting unit 21, the medical interview answer data acquiring unit 13 presents, to the user terminal 100, data on an additional medical interview sheet demanding the detailed answer related to the disease extracted as the disease candidate and acquires medical interview answer data to the additional medical interview sheet.

In this case, the affection probability analyzing unit 18 analyses the affection probability again including the medical interview answer data acquired additionally by the medical interview answer data. In the case in which the affection probability obtained by the re-analysis through the affection probability analyzing unit 18 is equal to or greater than a first threshold, then, the disease candidate extracting unit 21 changes the disease extracted as the "disease doubt" into the "disease positivity". Such processing is repeated by the number of disease candidates extracted as the "disease doubt".

By providing the additional function, it is possible to decrease an oversight risk in the disease candidate to be the "disease positivity".

Although the description has been given to the example in which the risk ratio is used for calculating the affection probability P in the embodiment, moreover, it is also possible to use an odds ratio (an index indicative of the number of more factors of a disease possessed by a sick person as compared with a person having no factor). In this case, the weight value calculated by the weight value calculating unit 23 also represents the odds ratio.

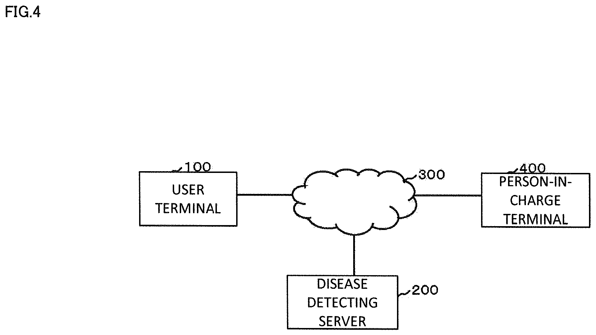

Although the description has been given to the structure in which the user terminal 100 and the disease detecting server 200 are connected to each other through the internet 300 in the embodiment, moreover, the present invention is not restricted thereto. For example, as shown in FIG. 4, a person-in-charge terminal 400 to be used by an industrial physician or a person in charge of a company may further be provided, and the detection result presenting unit 22 may present, to the person-in-charge terminal 400, a result obtained by detecting the disease through the disease detecting server 200 based on an answer input by using the user terminal 100 through an employee of a company or the like.

Although the description has been given to the example in which the free answer data acquiring unit 14 of the disease detecting server 200 presents, to the user terminal 100, data on a question which can be answered in a free sentence and acquires the answer of the user to the question as free answer data in a text sentence in the embodiment, moreover, the present invention is not restricted thereto. For example, the free answer data may be acquired by exchange on an interactive basis using a voice.

For example, voice data on a question which can be answered freely is transmitted from the disease detecting server 200 to the user terminal 100 and outputs the question in a voice from a speaker of the user terminal 100. Then, a speaker voice in which the user answered to the question may be input from a microphone and transmitted as voice data to the disease detecting server 200, and may be subjected to voice recognition and be converted into free answer data in a text sentence.

In this case, when a question of "What is hard ?" in a voice is given from the disease detecting server 200 to the user terminal 100 and the user answers to the question, for example, "I have many cares and cannot sleep at night", the disease detecting server 200 extracts a constitution word such as "cares" or "cannot sleep at night" from the answer voice and extracts a disease candidate, for example, a neurosis, an adjustment disorder, a depression or the like therefrom. Furthermore, a next additional question, that is, "That's too bad. Is there a trouble in your life ? Do you get depressed ?" can be successively given depending on the extracted disease candidate and the disease candidate can be narrowed down corresponding to the content of an answer to the additional question. Moreover, it is also possible to store data input through a serial interaction in the actual result database 17 and to increase the precision in the weight value (risk ratio) to be calculated by the weight value calculating unit 23 after the end of the session.

In addition, the embodiment is only illustrative for concreteness to carry out the present invention and the technical scope of the present invention is defined by the appended claims. In other words, the present invention can be carried out in various configurations without departing from the scope of the claims.

**EXPLANATION OF DESIGNATION**

| | |
|---|---|
| 12 | profile data acquiring unit |
| 13 | medical interview answer data acquiring unit |
| 14 | free answer data acquiring unit |
| 15 | free sentence analyzing unit |
| 16 | database storing unit |
| 17 | actual result database |
| 18 | affection probability analyzing unit |
| 19 | disease database |
| 20 | weight value storing unit |
| 21 | disease candidate extracting unit |
| 22 | detection result presenting unit |
| 23 | weight value calculating unit |
| 100 | user terminal |
| 200 | disease detecting server |

**Claims**

1.   A disease detecting system comprising:

a weight value storing unit for storing, as a weight value, a risk ratio to be an index indicative of a liability to a disease of a person having a factor of the disease as compared with a person having no factor;
a disease database prestoring, as various parameters, a prevalence for each disease, a sensitivity and specificity for each disease to each question which is included in a medical interview sheet, a risk ratio for each disease, and a profile element related thereto;
a profile data acquiring unit for acquiring profile data of a user;
a medical interview answer data acquiring unit for presenting data, to the user, on the medical interview sheet including an open question in which a sensitivity and a specificity of a disease are known and a close question in which a risk ratio of the disease is known and acquiring medical interview answer data to be an answer to the medical interview sheet;
a free answer data acquiring unit for presenting, to the user, data on a question in which a subjective symptom can be answered in a free sentence, and acquiring free answer data to be an answer to the question;
a free sentence analyzing unit for analyzing the free answer data acquired by the free answer data acquiring unit and generating, as text related data, data on at least one of a constitution word, word segmentation, a segment, information about a distance between words and text dependency information;
a database storing unit for storing, in an actual result database, the profile data acquired by the profile data acquiring unit, the medical interview answer data acquired by the medical interview answer data acquiring unit, and the text related data generated by the free sentence analyzing unit;
an affection probability analyzing unit for analyzing an affection probability representing a possibility that the user might be affected with a specific disease by using the various parameters stored for each disease in the disease database, the profile data acquired by the profile data acquiring unit, the medical interview answer data acquired by the medical interview answer data acquiring unit, the text related data generated by the free sentence analyzing unit, and the weight value stored in the weight value storing unit;
a disease candidate extracting unit for extracting, as a disease candidate, a disease having the affection probability obtained by the affection probability analyzing unit which is equal to or greater than a threshold; and
a weight value calculating unit for setting, as explanatory variables, the profile data, the medical interview answer data and the text related data which are stored in the actual result database and setting, as a criterion variable, the disease candidate extracted by the disease candidate extracting unit to perform regression analysis, thereby calculating the weight value for the explanatory variable serving as a factor to be correlated with the disease candidate extracted by the disease candidate extracting unit and updating and storing the weight value in the weight value storing unit.

2. The disease detecting system according to claim 1, wherein the disease candidate extracting unit extracts, as a disease candidate for a disease positivity, a disease having the affection probability obtained by the affection probability analyzing unit which is equal to or greater than a first threshold, while it extracts, as a disease candidate for a disease doubt, a disease having the affection probability which is smaller than the first threshold and is equal to or greater than a second threshold.

3. The disease detecting system according to claim 2, wherein when the disease candidate for the disease positivity is extracted by the disease candidate extracting unit, the free answer data acquiring unit presents, to the user, data on an additional question for demanding a detailed answer in a free sentence related to the disease extracted as the disease candidate, and acquires free answer data to the additional question,
the free sentence analyzing unit further analyzes the additional free answer data acquired by the free answer data acquiring unit to generate the text related data, and
the database storing unit further stores, in the actual result database, the text related data generated additionally by the free sentence analyzing unit.

4. The disease detecting system according to claim 2, wherein when the disease candidate for the disease doubt is extracted by the disease candidate extracting unit, the medical interview answer data acquiring unit presents, to the user, data on an additional medical interview sheet demanding a detailed answer related to the disease extracted as the disease candidate and acquires medical interview answer data for the additional medical interview sheet,
the affection probability analyzing unit re-analyzes the affection probability including the medical interview answer data acquired additionally through the medical interview answer data, and
the disease candidate extracting unit changes the disease extracted as the disease doubt into the disease positivity when the affection probability obtained by the re-analysis of the affection probability analyzing unit is equal to or greater than the first threshold.

5. The disease detecting system according to claim 1, wherein the affection probability analyzing unit calculates a pre-examination probability by multiplying a known prevalence of a disease by a risk ratio corresponding to a profile based on the profile data acquired by the profile data acquiring unit and the various parameters stored for each disease in the disease database,
calculates a cumulative risk ratio based on the medical interview answer data acquired by the medical interview answer data acquiring unit, the text related data generated by the free sentence analyzing unit, and the various parameters stored for each disease in the disease database, and
calculates the affection probability by multiplying the pre-examination probability by the cumulative risk ratio.

6. A: computer implemented method of detecting a possibility that a user might be affected with a specific disease in a disease detecting system including a weight value storing unit for storing, as a weight value, a risk ratio to be an index indicative of a liability to a disease of a person having a factor of the disease as compared with a person having no factor and a disease database prestoring, as various parameters, a prevalence for each disease, a sensitivity and specificity for each disease or each question which is included in a medical interview sheet, a risk ratio for each disease, and a profile element related thereto, the method comprising:

a first step of causing a profile data acquiring unit of a disease detecting system to acquire profile data of a user;
a second step of causing a medical interview answer data acquiring unit of the disease detecting system to present data on the medical interview sheet including an open question in which a sensitivity and a specificity of a disease are known and a close question in which a risk ratio of the disease is known to the user and to acquire medical interview answer data to be an answer to the medical interview sheet;
a third step of causing a free answer data acquiring unit of the disease detecting system to present, to the user, data on a question which a subjective symptom can be answered in a free sentence, and to acquire free answer data to be an answer to the question;
a fourth step of causing a free sentence analyzing unit of the disease detecting system to analyze the free answer data acquired by the free answer data acquiring unit and to generate, text related data, data on at least one of a constitution word, word segmentation, a segment, information about a distance between words and text dependency information;
a fifth step of causing a database storing unit of the disease detecting system to store, in an actual result database, the profile data acquired by the profile data acquiring unit, the medical interview answer data acquired by the medical interview answer data acquiring unit, and the text related data generated by the free sentence analyzing unit;

a sixth step of causing an affection probability analyzing unit of the disease detecting system to analyze an affection probability representing a possibility that the user might be affected with a specific disease by using the various parameters stored for each disease in the disease database, the profile data acquired by the profile data acquiring unit, the medical interview answer data acquired by the medical interview answer data acquiring unit, the text related data generated by the free sentence analyzing unit, and the weight value stored in the weight value storing unit;

a seventh step of causing a disease candidate extracting unit of the disease detecting system to extract, as a disease candidate, a disease having the affection probability obtained by the affection probability analyzing unit which is equal to or greater than a threshold; and

an eighth step of causing a weight value calculating unit of the disease detecting system to set, as explanatory variables, the profile data, the medical interview answer data and the text related data which are stored in the actual result database and setting, as a criterion variable, the disease candidate extracted by the disease candidate extracting unit, thereby performing regression analysis to calculate the weight value for the explanatory variable serving as a factor to be correlated with the disease candidate extracted by the disease candidate extracting unit and to update and store the weight value in the weight value storing unit.

## Patentansprüche

1. Krankheitserkennungssystem, das aufweist:

eine Gewichtswert-Speichereinheit zum Speichern als einen Gewichtswert eines Risikoverhältnisses, der ein Index sein soll, der eine Anfälligkeit gegenüber einer Krankheit einer Person, die einen Faktor der Krankheit aufweist, im Vergleich zu einer Person anzeigt, die keinen Faktor aufweist;

eine Krankheitsdatenbank, die als verschiedene Parameter eine Prävalenz für jede Krankheit, eine Empfindlichkeit und Spezifität für jede Krankheit oder jede Frage, die in einem medizinischen Fragebogen enthalten ist, ein Risikoverhältnis für jede Krankheit und ein damit zusammenhängendes Profilelement vorspeichert;

eine Profildaten-Erfassungseinheit zum Erfassen von Profildaten eines Benutzers;

eine Einheit zur Erfassung medizinischer Befragungsantwortdaten, um dem Benutzer Daten auf dem medizinischen Fragebogen zu präsentieren, die eine offene Frage, in der eine Empfindlichkeit und eine Spezifität auf eine Krankheit bekannt sind, und eine geschlossene Frage aufweisen, in der ein Risikoverhältnis der Krankheit bekannt ist, und medizinische Befragungsantwortdaten zu erfassen, die eine Antwort auf den medizinischen Fragebogen sein sollen;

eine Einheit zur Erfassung freier Antwortdaten, um dem Benutzer Daten zu einer Frage zu präsentieren, in der ein subjektives Symptom in einem freien Satz beantwortet werden kann, und freie Antwortdaten zu erfassen, die eine Antwort auf die Frage sein sollen;

eine Einheit zu Analyse freier Sätze zum Analysieren der durch die Einheit zur Erfassung freier Antwortdaten erfassten freien Antwortdaten und zum Erzeugen als textbezogene Daten von Daten über ein Zusammensetzungswort und/oder eine Wortsegmentierung und/oder ein Segment und/oder Informationen über einen Abstand zwischen Wörtern und/oder Textabhängigkeitsinformationen;

eine Datenbank-Speichereinheit zum Speichern in einer Datenbank tatsächlicher Ergebnisse der durch die Profildaten-Erfassungseinheit erfassten Profildaten, der durch die Einheit zur Erfassung medizinischer Befragungsantwortdaten erfassten medizinischen Befragungsantwortdaten und der durch die Einheit zur Analyse freier Sätze erzeugten textbezogenen Daten;

eine Erkrankungswahrscheinlichkeits-Analyseeinheit zum Analysieren einer Erkrankungswahrscheinlichkeit, die eine Wahrscheinlichkeit repräsentiert, dass der Benutzer durch eine spezifische Krankheit betroffen sein könnte, unter Verwendung der für jede Krankheit in der Krankheitsdatenbank gespeicherten verschiedenen Parameter, der durch die Profildaten-Erfassungseinheit erfassten Profildaten, der durch die Einheit zur Erfassung medizinischer Befragungsantwortdaten erfassten medizinischen Befragungsantwortdaten, der durch die Einheit zur Analyse freier Sätze erzeugten textbezogenen Daten und des in der Gewichtswert-Speichereinheit gespeicherten Gewichtswerts;

eine Krankheitskandidaten-Extraktionseinheit zum Extrahieren als einen Krankheitskandidaten einer Krankheit, die die durch die Erkrankungswahrscheinlichkeits-Analyseeinheit erhaltene Erkrankungswahrscheinlichkeit aufweist, die größer oder gleich einem Schwellenwert ist; und

eine Gewichtswert-Berechnungseinheit zum Setzen als erklärende Variablen der Profildaten, der medizinischen Befragungsantwortdaten und der textbezogenen Daten, die in der Datenbank tatsächlicher Ergebnisse gespeichert sind, und zum Setzen als eine Kriterienvariable der durch die Krankheitskandidaten-Extraktionseinheit extrahierten Krankheitskandidaten, um eine Regressionsanalyse durchzuführen, wodurch der Gewichtswert für die erklärende Variable berechnet wird, der als ein Faktor dient, der mit den durch die Krankheitskandidaten-Extraktionseinheit extrahierten Krankheitskandidaten korreliert werden soll, und Aktualisieren und Speichern des Gewichtswerts in der Gewichtswert-Speichereinheit.

2. Krankheitserkennungssystem nach Anspruch 1, wobei die Krankheitskandidaten-Extraktionseinheit als einen Krankheitskandidaten für eine Krankheitspositivität eine Krankheit extrahiert, die die durch die Erkrankungswahrscheinlichkeits-Analyseeinheit erhaltene Erkrankungswahrscheinlichkeit aufweist, die gleich oder höher als ein erster Schwellenwert ist, während sie als einen Krankheitskandidaten für einen Krankheitszweifel eine Krankheit extrahiert, die die Erkrankungswahrscheinlichkeit aufweist, die kleiner als der erste Schwellenwert ist und gleich oder höher als ein zweiter Schwellenwert ist.

3. Krankheitserkennungssystem nach Anspruch 2, wobei wenn die Krankheitskandidaten für die Krankheitspositivität durch die Krankheitskandidaten-Extraktionseinheit extrahiert werden, die Einheit zur Erfassung freier Antwortdaten dem Benutzer Daten über eine zusätzliche Frage zum Erfragen einer detaillierten Antwort in einem freien Satz präsentiert, die die als den Krankheitskandidaten extrahierte Krankheit betrifft, und freie Antwortdaten zu der zusätzlichen Frage erfasst,
die Einheit zu Analyse freier Sätze ferner die zusätzlichen durch die Einheit zur Erfassung freier Antwortdaten erfassten freien Antwortdaten analysiert, um die textbezogenen Daten zu erzeugen, und
die Datenbank-Speichereinheit ferner in der Datenbank tatsächlicher Ergebnisse die textbezogenen Daten speichert, die durch die Einheit zur Analyse freier Sätze zusätzlich erzeugt werden.

4. Krankheitserkennungssystem nach Anspruch 2, wobei wenn die Krankheitskandidaten für den Krankheitszweifel durch die Krankheitskandidaten-Extraktionseinheit extrahiert werden, die Einheit zur Erfassung medizinischer Befragungsantwortdaten dem Benutzer Daten über einen zusätzlichen medizinischen Fragebogen präsentiert, der eine detaillierte Antwort erfragt, die die als den Krankheitskandidaten extrahierte Krankheit betrifft, und medizinische Befragungsantwortdaten für den zusätzlichen medizinischen Fragebogen erfasst,
die Erkrankungswahrscheinlichkeits-Analyseeinheit die Erkrankungswahrscheinlichkeit einschließlich der medizinischen Befragungsantwortdaten erneut analysiert, die durch die medizinischen Befragungsantwortdaten zusätzlich erfasst wurden, und
die Krankheitskandidaten-Extraktionseinheit die als der Krankheitszweifel extrahierte Krankheit in die Krankheitspositivität ändert, wenn die durch die erneute Analyse der Erkrankungswahrscheinlichkeits-Analyseeinheit erhaltene Erkrankungswahrscheinlichkeit gleich oder höher als der erste Schwellenwert ist.

5. Krankheitserkennungssystem nach Anspruch 1, wobei die Erkrankungswahrscheinlichkeits-Analyseeinheit eine Voruntersuchungswahrscheinlichkeit durch Multiplizieren einer bekannten Prävalenz einer Krankheit mit einem Risikoverhältnis, das einem Profil entspricht, beruhend auf den durch die Profildaten-Erfassungseinheit erfassten Profildaten und den für jede Krankheit in der Krankheitsdatenbank gespeicherten verschiedenen Parametern berechnet,
ein kumulatives Risikoverhältnis beruhend auf den durch die Einheit zur Erfassung medizinischer Befragungsantwortdaten erfassten medizinischen Befragungsantwortdaten, den durch die Einheit zur Analyse freier Sätze erzeugten textbezogenen Daten und den für jede Krankheit in der Krankheitsdatenbank gespeicherten verschiedenen Parametern berechnet, und
die Erkrankungswahrscheinlichkeit durch Multiplizieren der Voruntersuchungswahrscheinlichkeit mit dem kumulativen Risikoverhältnis berechnet.

6. Computerimplementiertes Verfahren zum Erkennen einer Wahrscheinlichkeit, dass ein Benutzer durch eine spezifische Krankheit betroffen sein könnte, in einem Krankheitserkennungssystem, das eine Gewichtswert-Speichereinheit zum Speichern als einen Gewichtswert eines Risikoverhältnisses, der ein Index sein soll, der eine Anfälligkeit gegenüber einer Krankheit einer Person, die einen Faktor der Krankheit aufweist, im Vergleich zu einer Person anzeigt, die keinen Faktor aufweist, und eine Krankheitsdatenbank aufweist, die als verschiedene Parameter eine Prävalenz für jede Krankheit, eine Empfindlichkeit und Spezifität für jede Krankheit oder jede Frage, die in einem medizinischen Fragebogen enthalten ist, ein Risikoverhältnis für jede Krankheit und ein damit zusammenhängendes Profilelement vorspeichert, wobei das Verfahren aufweist:

einen ersten Schritt zum Veranlassen einer Profildaten-Erfassungseinheit eines Krankheitserkennungssystems, Profildaten eines Benutzers zu erfassen;

einen zweiten Schritt zum Veranlassen einer Einheit zur Erfassung medizinischer Befragungsantwortdaten des Krankheitserkennungssystems, Daten auf dem medizinischen Fragebogen, die eine offene Frage, in der eine Empfindlichkeit und eine Spezifität auf eine Krankheit bekannt sind, und eine geschlossene Frage aufweisen, in der ein Risikoverhältnis der Krankheit bekannt ist, dem Benutzer zu präsentieren und medizinische Befragungsantwortdaten zu erfassen, die eine Antwort auf den medizinischen Fragebogen sein sollen;

einen dritten Schritt zum Veranlassen einer Einheit zur Erfassung freier Antwortdaten des Krankheitserkennungssystems, dem Benutzer Daten zu einer Frage zu präsentieren, in der ein subjektives Symptom in einem freien Satz beantwortet werden kann, und freie Antwortdaten zu erfassen, die eine Antwort auf die Frage sein sollen;

einen vierten Schritt zum Veranlassen einer Einheit zur Analyse freier Sätze des Krankheitserkennungssystems, die durch die Einheit zur Erfassung freier Antwortdaten erfassten freien Antwortdaten zu analysieren und textbezogene Daten, Daten über ein Zusammensetzungswort, eine Wortsegmentierung, ein Segment, Informationen über einen Abstand zwischen Wörtern und Textabhängigkeitsinformationen zu erzeugen;

einen fünften Schritt zum Veranlassen einer Datenbank-Speichereinheit des Krankheitserkennungssystems in einer Datenbank tatsächlicher Ergebnisse die durch die Profildaten-Erfassungseinheit erfassten Profildaten, die durch die Einheit zur Erfassung medizinischer Befragungsantwortdaten erfassten medizinischen Befragungsantwortdaten und die durch die Einheit zur Analyse freier Sätze erzeugten textbezogenen Daten zu speichern;

einen sechsten Schritt zum Veranlassen einer Erkrankungswahrscheinlichkeits-Analyseeinheit des Krankheitserkennungssystems, eine Erkrankungswahrscheinlichkeit, die eine Wahrscheinlichkeit repräsentiert, dass der Benutzer durch eine spezifische Krankheit betroffen sein könnte, unter Verwendung der für jede Krankheit in der Krankheitsdatenbank gespeicherten verschiedenen Parameter, der durch die Profildaten-Erfassungseinheit erfassten Profildaten, der durch die Einheit zur Erfassung medizinischer Befragungsantwortdaten erfassten medizinischen Befragungsantwortdaten, der durch die Einheit zur Analyse freier Sätze erzeugten textbezogenen Daten und der in der Gewichtswert-Speichereinheit gespeicherten Gewichtswerts zu analysieren;

einen siebenten Schritt zum Veranlassen einer Krankheitskandidaten-Extraktionseinheit des Krankheitserkennungssystems als einen Krankheitskandidaten eine Krankheit zu extrahieren, die die durch die Erkrankungswahrscheinlichkeits-Analyseeinheit erhaltene Erkrankungswahrscheinlichkeit aufweist, die größer oder gleich einem Schwellenwert ist; und

einen achten Schritt zum Veranlassen einer Gewichtswert-Berechnungseinheit des Krankheitserkennungssystems als erklärende Variablen die Profildaten, die medizinischen Befragungsantwortdaten und die textbezogenen Daten, die in der Datenbank tatsächlicher Ergebnisse gespeichert sind, zu setzen, und als eine Kriterienvariable die durch die Krankheitskandidaten-Extraktionseinheit extrahierten Krankheitskandidaten zu setzen, wodurch eine Regressionsanalyse durchgeführt wird, um den Gewichtswert für die erklärende Variable zu berechnen, der als ein Faktor dient, der mit den durch die Krankheitskandidaten-Extraktionseinheit extrahierten Krankheitskandidaten korreliert werden soll, und den Gewichtswert in der Gewichtswert-Speichereinheit zu aktualisieren und zu speichern.

## Revendications

1. Système de détection de maladie, comprenant :

une unité de mémorisation de valeur pondérale destinée à enregistrer en tant que valeur pondérale un risque relatif comme indice indicatif d'une susceptibilité à une maladie d'une personne présentant un facteur de la maladie comparativement à une personne dépourvue de facteur ;

une base de données de maladies pré-enregistrant, en tant que différents paramètres, une prévalence pour chaque maladie, une sensibilité et une spécificité pour chaque maladie en réponse à chaque question comprise dans une fiche de consultation médicale, un risque relatif pour chaque maladie, et un élément de profil correspondant ;

une unité d'acquisition de données de profil destinée à acquérir des données de profil d'un utilisateur ;

une unité d'acquisition de données de réponses à des questions de diagnostic destinée à présenter à l'utilisateur des données sur la fiche de consultation médicale comprenant une question ouverte où une sensibilité et une spécificité d'une maladie sont connues et une question fermée où un risque relatif de la maladie est connu, et à acquérir des données de réponses à des questions de diagnostic comme réponse à la fiche de consultation médicale ;

une unité d'acquisition de données de libre réponse destinée à présenter à l'utilisateur des données sur une question où un symptôme subjectif peut faire l'objet d'une réponse dans une libre déclaration, et à acquérir des données de libre réponse comme réponse à la question ;

une unité d'analyse de libre déclaration destinée à analyser les données de libre réponse acquises par l'unité d'acquisition de données de libre réponse et à générer en tant que données relatives à un texte, des données sur un mot de constitution et/ou une segmentation de mot et/ou un segment et/ou des informations sur une distance entre mots et informations de dépendance du texte ;

un unité d'enregistrement de base de données destinée à enregistrer, dans une base de données de résultats actuels, les données de profil acquises par l'unité d'acquisition de données de profil, les données de réponses à des questions de diagnostic acquises par l'unité d'acquisition de données de réponses à des questions de diagnostic, et les données relatives à un texte générées par l'unité d'analyse de libre déclaration ;

une unité d'analyse de probabilité d'affection destinée à analyser une probabilité d'affection représentant une possibilité que l'utilisateur puisse être affecté d'une maladie spécifique au moyen des différents paramètres enregistrés pour chaque maladie dans la base de données de maladies, des données de profil acquises par l'unité d'acquisition de données de profil, des données de réponses à des questions de diagnostic acquises par l'unité d'acquisition de données de réponses à des questions de diagnostic, des données relatives à un texte générées par l'unité d'analyse de libre déclaration, et de la valeur pondérale enregistrée dans l'unité de mémorisation de valeur pondérale ;

une unité d'extraction de maladie candidate destinée à extraire en tant que maladie candidate, une maladie dont la probabilité d'affection obtenue par l'unité d'analyse de probabilité d'affection est égale ou supérieure à un seuil ; et

une unité de calcul de valeur pondérale destiné à fixer en tant que variables explicatives, les données de profil, les données de réponses à des questions de diagnostic et les données relatives à un texte enregistrées dans la base de données de résultats actuels et à fixer en tant que variable de critère, la maladie candidate extraite par l'unité d'extraction de maladie candidate pour effectuer une analyse de régression, en calculant ainsi la valeur pondérale pour la variable explicative servant de facteur à corréler à la maladie candidate extraite par l'unité d'extraction de maladie candidate, et en actualisant et en enregistrant la valeur pondérale dans l'unité de mémorisation de valeur pondérale.

2. Système de détection de maladie selon la revendication 1, où l'unité d'extraction de maladie candidate extrait en tant que maladie candidate pour une positivité à la maladie, une maladie dont la probabilité d'affection obtenue par l'unité d'analyse de probabilité d'affection est égale ou supérieure à un premier seuil en extrayant, en tant que maladie candidate pour une suspicion de maladie, une maladie dont la probabilité d'affection est inférieure au premier seuil et égale ou supérieure à un deuxième seuil.

3. Système de détection de maladie selon la revendication 2, où, quand la maladie candidate pour la positivité à la maladie est extraite par l'unité d'extraction de maladie candidate, l'unité d'acquisition de données de libre réponse présente à l'utilisateur des données sur une question additionnelle pour demander une réponse détaillée dans une libre déclaration relative à la maladie extraite en tant que maladie candidate, et acquiert des données de libre réponse à la question additionnelle,

l'unité d'analyse de libre déclaration analyse en outre les données de libre réponse additionnelle acquises par l'unité d'acquisition de données de libre réponse pour générer les données relatives à un texte, et

l'unité d'enregistrement de base de données enregistre en outre dans la base de données de résultats actuels, les données relatives à un texte générées additionnellement par l'unité d'analyse de libre déclaration.

4. Système de détection de maladie selon la revendication 2, où, quand la maladie candidate pour la suspicion de maladie est extraite par l'unité d'extraction de maladie candidate, l'unité d'acquisition de données de réponses à des questions de diagnostic présente à l'utilisateur des données sur une fiche de consultation médicale additionnelle demandant une réponse détaillée relative à la maladie extraite en tant que maladie candidate, et acquiert des données de réponses à des questions de diagnostic pour la fiche de consultation médicale additionnelle,

l'unité d'analyse de probabilité d'affection analyse à nouveau la probabilité d'affection comprenant les données de réponses à des questions de diagnostic acquises additionnellement par les données de réponses à des questions de diagnostic, et

l'unité d'extraction de maladie candidate change la maladie extraite en tant que suspicion de maladie en positivité à la maladie si la probabilité d'affection obtenue la nouvelle analyse de l'unité d'analyse de probabilité d'affection est égale ou supérieure au premier seuil.

5. Système de détection de maladie selon la revendication 1, où l'unité d'analyse de probabilité d'affection calcule une probabilité d'examen préliminaire par multiplication d'une prévalence connue d'une maladie par un risque relatif correspondant à un profil sur la base des données de profil acquises par l'unité d'acquisition de données de profil et des différents paramètres enregistrés pour chaque maladie dans la base de données de maladies, calcule un risque relatif cumulé sur la base des données de réponses à des questions de diagnostic acquises par l'unité d'acquisition de données de réponses à des questions de diagnostic, des données relatives à un texte générées par l'unité d'analyse de libre déclaration, et des différents paramètres enregistrés pour chaque maladie dans la base de données de maladies, et

calcule la probabilité d'affection par multiplication de la probabilité d'examen préliminaire par le risque relatif cumulé.

6. Procédé informatique de détection d'une possibilité qu'un utilisateur puisse être affecté d'une maladie spécifique dans un système de détection de maladie comprenant une unité de mémorisation de valeur pondérale destinée à enregistrer en tant que valeur pondérale un risque relatif comme indice indicatif d'une susceptibilité à une maladie d'une personne présentant un facteur de la maladie comparativement à une personne dépourvue de facteur et une base de données de maladies pré-enregistrant, en tant que différents paramètres, une prévalence pour chaque maladie, une sensibilité et une spécificité pour chaque maladie en réponse à chaque question comprise dans une fiche de consultation médicale, un risque relatif pour chaque maladie, et un élément de profil correspondant, ledit procédé comprenant :

une première étape entraînant une unité d'acquisition de données de profil de système de détection de maladie à acquérir des données de profil d'un utilisateur ;

une deuxième étape entraînant une unité d'acquisition de données de réponses à des questions de diagnostic de système de détection de maladie à présenter à l'utilisateur des données sur la fiche de consultation médicale comprenant une question ouverte où une sensibilité et une spécificité d'une maladie sont connues et une question fermée où un risque relatif de la maladie à connu et à acquérir des données de réponses à des questions de diagnostic comme réponse à la fiche de consultation médicale ;

une troisième étape entraînant une unité d'acquisition de données de libre réponse de système de détection de maladie à présenter à l'utilisateur des données sur une question où un symptôme subjectif peut faire l'objet d'une réponse dans une libre déclaration, et à acquérir des données de libre réponse comme réponse à la question ;

une quatrième étape entraînant une unité d'analyse de libre déclaration de système de détection de maladie à analyser les données de libre réponse acquises par l'unité d'acquisition de données de libre réponse et à générer en tant que données relatives à un texte, des données sur un mot de constitution et/ou une segmentation de mot et/ou un segment et/ou des informations sur une distance entre mots et informations de dépendance du texte ;

une cinquième étape entraînant une unité d'enregistrement de base de données de système de détection de maladie à enregistrer, dans une base de données de résultats actuels, les données de profil acquises par l'unité d'acquisition de données de profil, les données de réponses à des questions de diagnostic acquises par l'unité d'acquisition de données de réponses à des questions de diagnostic, et les données relatives à un texte générées par l'unité d'analyse de libre déclaration ;

une sixième étape entraînant une unité d'analyse de probabilité d'affection de système de détection de maladie à analyser une probabilité d'affection représentant une possibilité que l'utilisateur puisse être affecté d'une maladie spécifique au moyen des différents paramètres enregistrés pour chaque maladie dans la base de données de maladies, des données de profil acquises par l'unité d'acquisition de données de profil, des données de réponses à des questions de diagnostic acquises par l'unité d'acquisition de données de réponses à des questions de diagnostic, des données relatives à un texte générées par l'unité d'analyse de libre déclaration, et de la valeur pondérale enregistrée dans l'unité de mémorisation de valeur pondérale ;

une septième étape entraînant une unité d'extraction de maladie candidate de système de détection de maladie à extraire en tant que maladie candidate une maladie dont la probabilité d'affection obtenue par l'unité d'analyse de probabilité d'affection est égale ou supérieure à un seuil ; et

une huitième étape entraînant une unité de calcul de valeur pondérale de système de détection de maladie à fixer en tant que variables explicatives, les données de profil, les données de réponses à des questions de diagnostic et les données relatives à un texte qui sont enregistrées dans la base de données de résultats actuels et à fixer en tant que variable de critère, la maladie candidate extraite par l'unité d'extraction de maladie candidate, en effectuant ainsi une analyse de régression pour calculer la valeur pondérale pour la variable explicative servant de facteur à corréler à la maladie candidate extraite par l'unité d'extraction de maladie candidate, et pour actualiser et enregistrer la valeur pondérale dans l'unité de mémorisation de valeur pondérale.

FIG.1

```
   ⌒100                 ⌒300              ⌒200
┌──────────┐                          ┌──────────┐
│  USER    │      ⌒⌒⌒⌒⌒⌒⌒            │ DISEASE  │
│          │─────(           )────────│ DETECTING│
│ TERMINAL │      ⌒⌒⌒⌒⌒⌒⌒            │  SERVER  │
└──────────┘                          └──────────┘
```

FIG.2

FIG.3

(a)

```
          ┌─────────────┐
          │    START    │
          └─────────────┘
                 │
                 ▼
┌──────────────────────────────────┐
│      ACQUIRE PROFILE DATA         │ ～ S1
└──────────────────────────────────┘
                 │
                 ▼
┌──────────────────────────────────┐
│ ACQUIRE MEDICAL INTERVIEW ANSWER DATA │ ～ S2
└──────────────────────────────────┘
                 │
                 ▼
┌──────────────────────────────────┐
│      ACQUIRE FREE ANSWER DATA     │ ～ S3
└──────────────────────────────────┘
                 │
                 ▼
┌──────────────────────────────────┐
│       ANALYZE FREE SENTENCE       │ ～ S4
└──────────────────────────────────┘
                 │
                 ▼
┌──────────────────────────────────┐
│ STORE PROFILE DATA, MEDICAL INTERVIEW ANSWER DATA │ ～ S5
│      AND TEXT RELATED DATA IN DB  │
└──────────────────────────────────┘
                 │
                 ▼
┌──────────────────────────────────┐
│     ANALYZE AFFECTION PROBABILITY │ ～ S6
└──────────────────────────────────┘
                 │
                 ▼
┌──────────────────────────────────┐
│      EXTRACT AFFECTION CANDIDATE  │ ～ S7
└──────────────────────────────────┘
                 │
                 ▼
┌──────────────────────────────────┐
│ PRESENT EXTRACTED AFFECTION CANDIDATE │ ～ S8
└──────────────────────────────────┘
                 │
                 ▼
          ┌─────────────┐
          │     END     │
          └─────────────┘
```

(b)

```
          ┌─────────────┐
          │    START    │
          └─────────────┘
                 │
                 ▼
┌──────────────────────────────────┐
│ CALCULATE WEIGHT VALUE OF EXTRACTED AFFECTION │ ～ S9
│              CANDIDATE            │
└──────────────────────────────────┘
                 │
                 ▼
┌──────────────────────────────────┐
│    STORE CALCULATED WEIGHT VALUE  │ ～ S10
└──────────────────────────────────┘
                 │
                 ▼
          ┌─────────────┐
          │     END     │
          └─────────────┘
```

FIG.4

```
   ╭─100                            ╭─300          ╭─400
 ┌──────────┐          ☁☁☁☁☁        ┌──────────────┐
 │  USER    │       ☁☁     ☁☁       │ PERSON-IN-   │
 │ TERMINAL │──────☁          ☁─────│  CHARGE      │
 │          │       ☁☁     ☁☁       │ TERMINAL     │
 └──────────┘          ☁☁☁☁☁        └──────────────┘
                         │
                    ┌──────────┐
                    │ DISEASE  │  ╭─200
                    │DETECTING │
                    │ SERVER   │
                    └──────────┘
```

**EP 3 107 061 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008108199 A
- JP 2012179336 A
- JP HEI6176007 B
- US 20050137463 A1